# FASCICULE DE BREVET EUROPEEN

(11) **EP 0 617 975 B1**
(45) Date de publication et mention de la délivrance du brevet: **20.08.1997**
(21) Numéro de dépôt: 93400818.6
(22) Date de dépôt: 30.03.1993
(51) Int. Cl.: A61M 1/00

(54) **Valve de drainage implantable pour le traitement de l'hydrocéphalie**
Implantierbares Drainageventil zur Behandlung von Hydrocephalus
Implantable drainage valve for the treatment of hydrocephalus

(43) Date de publication de la demande: 05.10.1994
(73) Titulaire: CORDIS S.A., F-91170 Viry-Châtillon (FR)
(72) Inventeur: Lecuyer, Alain, F-06130 Grasse (FR)
(74) Mandataire: Bloch, Gérard

(56) Documents cités:
- EP-A- 0 060 369
- US-A- 3 889 687
- US-A- 4 673 384
- US-A- 5 042 974

## Description

La présente invention concerne une valve de drainage implantable pour le traitement de l'hydrocéphalie.

On connaît déjà divers types de valves destinées à contrôler le drainage d'un excès de fluide céphalo-rachidien contenu dans les ventricules cérébraux vers, par exemple, la cavité péritonéale.

C'est ainsi que l'on a déjà proposé d'utiliser de simples clapets régulateurs de pression qui s'ouvrent au-delà d'un certain seuil de pression différentielle entre leur orifice d'entrée et leur orifice de sortie, et qui empêchent ensuite cette pression différentielle de dépasser la valeur de seuil.

Ces valves, telles que décrites dans le document US-A-3889687, comportent un corps cylindrique ouvert à ses deux extrémités, l'orifice amont formant un siège pour une bille de fermeture. La bille est appliquée sur le siège par l'extrémité d'un ressort dont l'autre extrémité prend appuie à la périphérie de l'ouverture aval.

Ces valves présentent le sérieux inconvénient de ne pas tenir compte des différences de hauteur de colonne de liquide en fonction de la position, notamment debout ou couchée, du patient.

On a déjà proposé, notamment dans le document précité, de palier cet inconvénient en adjoignant au clapet un deuxième corps comportant un deuxième siège cônique sur lequel une bille est appliquée par l'effet de la gravité lorsque le dispositif est vertical, le dispositif présentant donc dans cette position une perte de charge supplémentaire compensant la pression artificielle dûe à la station debout.

Ces dispositifs impliquent toutefois l'usinage précis de deux sièges ce qui en augmente le coût.

En outre, la présence des deux corps les rend volumineux ce qui implique une implantation lombaire.

Enfin, la bille sur laquelle n'agit que la gravité introduit des effets dynamiques, ou balistiques, lors de mouvements saccadés telle que la course, effets dynamiques qui perturbent le bon fonctionnement du dispositif.

La présente invention vise à palier ces inconvénients.

A cet effet, l'invention a pour objet une valve de drainage implantable pour le traitement de l'hydrocéphalie comprenant un corps formant un siège de valve, un organe de fermeture et un ressort dont une extrémité applique l'organe de fermeture sur le siège, caractérisé par le fait qu'elle comprend une masselote sur laquelle s'appuie l'extrémité du ressort opposée à l'organe de fermeture.

Le corps peut être avantageusement cylindrique, la masselote comprenant au moins une bille logée dans le corps.

Une telle valve présente l'avantage qu'elle ne comporte qu'un seul siège et est par conséquent meilleur marché que les dispositifs connus.

Par ailleurs, les effets balistiques sont limités du fait de l'action du ressort sur la masselote. Enfin, cette valve présente un faible volume qui permet une implantation crânienne.

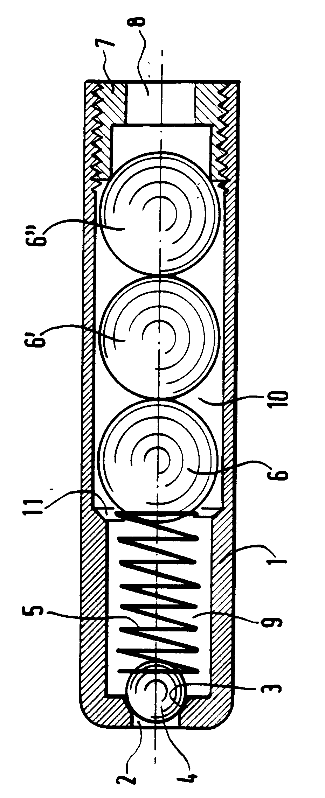

On décrira maintenant à titre d'exemple non limitatif un mode de réalisation particulier de l'invention en référence à la figure unique annexée représentant une vue en coupe axiale d'une valve selon l'invention.

Cette valve comporte un corps cylindrique 1 ouvert à ses deux extrémités. La périphérie de l'ouverture amont 2 forme un siège cônique 3 sur lequel une bille de fermeture 4 est appliquée par une extrémité d'un ressort hélicoïdal 5.

L'autre extrémité du ressort 5 est en appui sur une bille 6 formant masselote. Deux autres billes 6' et 6'' sont disposées dans le corps 1 où elles sont maintenues par une vis annulaire 7 formant en son centre l'ouverture aval 8 de la valve.

L'intérieur du corps 1 présente dans sa partie amont 9 occupée par le ressort 5 un diamètre plus faible que dans sa partie aval 10 occupée par les billes 6, 6' et 6'', de manière à former un épaulement 11 permettant en tout état de cause de limiter la course des billes 6.

En utilisation, l'ouverture amont 2 est raccordée à un cathéter dont l'extrémité libre est placée dans le ventricule cérébral à drainer, et l'ouverture aval 8 est raccordée à un ventricule de drainage dont l'extrémité libre est ramenée dans la cavité péritonéale.

Lorsque la valve est dans sa position horizontale représentée au dessin, la bille 4 n'est soumise qu'à la pression différentielle entre l'amont et l'aval et à la force exercée par le ressort 5.

Au contraire, en position verticale avec l'ouverture aval 8 orientée vers le haut, la bille 4 est soumise de plus au poids des billes 6 et a par conséquent tendance à être appliqué sur le siège 3 avec plus de force, compensant ainsi la hauteur de colonne de liquide correspondant à la station debout.

Tous les éléments de cette valve sont réunis dans un seul corps qui ne comporte qu'un seul siège d'ou un coût et un encombrement relativement limité.

Enfin, les billes 6 sont en permanence soumise à la force de réaction du ressort 5 ce qui limite grandement les effets dynamiques.

## Revendications

1. Valve de drainage implantable pour le traitement de l'hydrocéphalie, comprenant un corps (1) formant un siège (3) de valve, un organe de fermeture (4), et un ressort (5) dont une extrémité applique l'organe de fermeture sur le siège, caractérisé par le fait qu'elle comprend une masselote (6, 6', 6'') sur laquelle s'appuie l'extrémité du ressort opposée à l'organe de fermeture.

2. Valve selon la revendication 1, dans laquelle ledit corps est cylindrique, ladite masselote comprenant au moins une bille logée dans ledit corps.

3. Valve selon l'une quelconque des revendications 1 et 2, dans laquelle le ressort est un ressort hélicoïdal.

## Claims

1. An implantable drainage valve for the treatment of Hydrocephalus, comprising a body (1) forming a valve seat (3), a closure member (4), and a spring (5) of which one end urges the closure member against the seat, characterised by the fact that it includes a regulator means (6, 6', 6'') on which bears the end of the spring remote from the closure member.

2. A valve according to Claim 1, in which the body is cylindrical, the regulator means comprising at least one ball housed within the body.

3. A valve according to either of Claims 1 and 2, in which the spring is a helicoidal spring.

## Patentansprüche

1. Implantierbares Drainageventil zur Behandlung von Hydrocephalus mit einem Körper (1), welche einen Ventilsitz (3) bildet, einem Schließorgan (4) und einer Feder (5), deren eines Ende das Schließorgan zur Anlage gegen den Sitz beaufschlagt, **dadurch gekennzeichnet**, daß es eine Wirkmasse (6, 6', 6'') aufweist, welche das das vom Schließorgan abgewandte Ende der Feder beaufschlagt.

2. Ventil nach Anspruch 1, bei dem der Körper zylindrisch ausgebildet ist und die Wirkmasse wenigstens eine Kugel aufweist, welche in dem Körper aufgenommen ist.

3. Ventil nach einem der Ansprüche 1 oder 2, bei dem die Feder eine Schraubenfeder ist.
